# EUROPEAN PATENT APPLICATION

(11) **EP 4 548 902 A1**
(43) Date of publication of application: **07.05.2025**
(21) Application number: 23850062.3
(22) Date of filing: 31.07.2023
(51) Int. Cl.: A61K 6/30, A61K 6/60, A61K 6/62, A61K 6/71, A61K 6/887, A61K 6/896

(54) **KIT FOR PREPARING CURABLE COMPOSITION FOR DENTAL MATERIAL, CURABLE COMPOSITION FOR DENTAL MATERIAL, AND DENTAL MATERIAL**

(30) Priority: 03.08.2022 JP 2022124315
(71) Applicant: Mitsui Chemicals, Inc., Tokyo 104-0028 (JP)
(72) Inventor: DANJO, Takahiro, Sodegaura-shi, Chiba 299-0265 (JP); TAKAHASHI, Issei, Sodegaura-shi, Chiba 299-0265 (JP)
(74) Representative: Hoffmann Eitle
(86) International application number: PCT/JP2023/028028
(87) International publication number: WO 2024/029508

(57) **Abstract**

Provided is a kit for preparing a curable composition for a dental material including a polymerization accelerator, which includes a first preparation including a first monomer and a second preparation including a second monomer, in which at least one of the first preparation or the second preparation contains an organosiloxane including a polymerizable group and a hydroxyl or alkoxy group.

## Description

### Technical Field

The present disclosure relates to a kit for preparing a curable composition for a dental material, a curable composition for a dental material, and a dental material.

### BACKGROUND OF THE INVENTION

### Background Art

In the field of dentistry, synthetic resin moldings and the like are used to restore tooth defects. For example, curable compositions, so-called cements, are used as tooth substitutes for the restoration of large tooth defects. In recent years, the range of uses of cements has expanded.

Porcelain has come to be used for dental prostheses from an aesthetic point of view, and adhesion to not only tooth substance but also ceramics is required.

In the case of bonding ceramics using a curable composition for a dental material such as a cement, it is known to add a silane coupling agent to the composition for the purpose of improving the adhesive strength to the ceramics.

Patent Literature 1 describes a two-paste type dental curable composition composed of: a first preparation containing a polymerizable monomer having an acidic group (a), a polymerizable monomer not having an acidic group (b), a polymerization accelerator (c), and a filler (d); and a second preparation containing a polymerizable monomer not having an acidic group (b), at least one filler selected from the group consisting of lanthanum glass, barium glass, strontium glass, soda glass, zinc glass, fluoroaluminosilicate glass, and alumina (e), a polymerization accelerator (f), and a specified silane coupling agent (g).

Patent Literature 2 describes a two-paste type dental curable composition composed of: a first preparation containing a polymerizable monomer having an acidic group (a), a polymerizable monomer not having an acidic group (b), a polymerization accelerator (c), and a filler (d); and a second preparation containing a polymerizable monomer not having an acidic group (b), at least one basic filler selected from the group consisting of a basic glass filler and alumina (e), a polymerization accelerator (f), and a specified silane coupling agent (g), in which the first preparation further contains a specified silanol condensation catalyst (i) and/or the second preparation further contains a specified cross-linker (h).
Patent Literature 1: Japanese Patent Publication (JP-B) No. 7022749
Patent Literature 2: Japanese Patent Application Laid-Open (JP-A) No. 2019-94276

### SUMMARY OF THE INVENTION

### Technical Problem

As monomers used in cementitious materials, polyfunctional monomers having two or more polymerizable groups in one molecule are often used from the viewpoint of accelerating the curing rate and improving the strength of a cured product.

Meanwhile, silane coupling agents having a polymerizable group, such as those described in Patent Literature 1 or 2, may also be used for improving the adhesion to ceramics.

When such a silane coupling agent is added to a composition mainly containing the above-described polyfunctional monomer, the mechanical properties of the obtained cured product may deteriorate.

For these reasons, there is a demand for the production of a cured product that has excellent adhesion to ceramics and is inhibited from decreasing in mechanical properties.

An object for solving the problem in one embodiment of the disclosure is to provide a kit for preparing a curable composition for a dental material that allows obtaining a cured product with excellent adhesion to ceramics and reduced deterioration of mechanical properties, a curable composition for a dental material, and a dental material.

### Solution to Problem

Specific means for solving the problems include the following aspects.
<1> A kit for preparing a curable composition for a dental material including a polymerization accelerator, the kit including: a first preparation containing a first monomer; and a second preparation containing a second monomer, at least one of the first preparation or the second preparation contains an organosiloxane containing a polymerizable group and a hydroxyl or alkoxy group.
<2> The kit for preparing a curable composition for a dental material according to <1>, wherein the organosiloxane contains a compound represented by the following Formula (X-1).

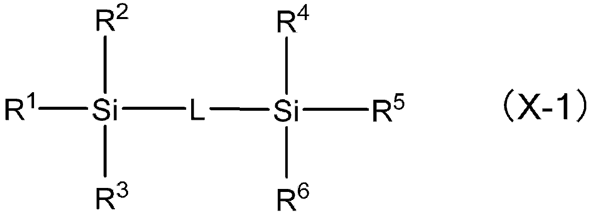

In Formula (X-1), R¹ to R⁶ each independently represent a monovalent organic group having a polymerizable group, a monovalent organic group not having a polymerizable group, or a hydroxyl group, and
L is an oxygen atom or a structure represented by the following Formula (Y-1).

Note that in Formulas (X-1) and (Y-1), at least one of R¹ to R⁸ is a monovalent organic group having a polymerizable group, and at least one of them is an alkoxy or hydroxyl group.

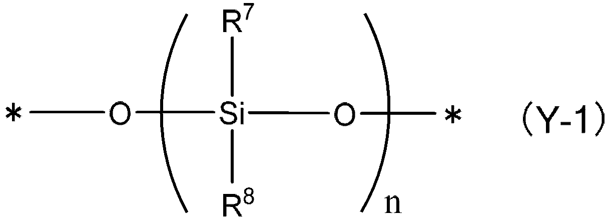

In Formula (Y-1), R⁷ to R⁸ each independently represent a monovalent organic group having a polymerizable group, a monovalent organic group not having a polymerizable group, a hydroxyl group, or an OSi chain, n is an integer of 1 or more, and * represents a bonding site.

The OSi chain is a monovalent organic group having an -O-Si group that forms a siloxane bond with an adjacent Si in Formula (Y-1).
<3> The kit for preparing a curable composition for a dental material according to <1> or <2>, wherein the organosiloxane has a molecular weight of from 200 to 1500.
<4> The kit for preparing a curable composition for a dental material according to any one of <1> to <3>, wherein the polymerization accelerator contains an oxidant and a reducing agent.
<5> The kit for preparing a curable composition for a dental material according to <4>, wherein the oxidant contains an organic peroxide, the reducing agent contains a reducing agent (A) that is a transition metal compound and a reducing agent (B) other than the transition metal compound, the first preparation contains the organic peroxide and the reducing agent (A), and the second preparation contains the reducing agent (B).
<6> The kit for preparing a curable composition for a dental material according to <5>, wherein the reducing agent (B) contains at least one selected from the group consisting of a thiourea, an ascorbic acid, a sulfinic acid, and an amine compound.
<7> The kit for preparing a curable composition for a dental material according to any one of <1> to <6>, wherein either one of the first monomer or the second monomer contains an acidic monomer.
<8> The kit for preparing a curable composition for a dental material according to <7>, wherein the first monomer contains the acidic monomer.
<9> The kit for preparing a curable composition for a dental material according to any one of <1> to <8>, wherein the first preparation and the second preparation contain a filler.
<10> The kit for preparing a curable composition for a dental material according to any one of <1> to <9>, wherein a total content of the organosiloxane contained in the first preparation and the second preparation is from 0.1% by mass to 15.0% by mass with respect to a total mass of a curable composition for a dental material that is to be prepared.
<11> A curable composition for a dental material, comprising a mixture of the first preparation and the second preparation of the kit for preparing a curable composition for a dental material according to any one of <1> to <10>.
<12> A dental material, comprising a cured product of the curable composition for a dental material according to <11>.

### Advantageous Effects of Invention

According to one embodiment of the disclosure, a kit for preparing a curable composition for a dental material that allows obtaining a cured product with excellent adhesion to ceramics and reduced deterioration of mechanical properties, a curable composition for a dental material, and a dental material can be provided.

### DESCRIPTION OF THE PREFERRED EMBODIMENTS

In the disclosure, a numerical range indicated using "to" means a range that includes the numerical values before and after "to" as the minimum and maximum values, respectively.

In the numerical ranges described in stages in the disclosure, the upper or lower limit value described in a certain numerical range may be replaced with the upper or lower limit value of another numerical range described in stages. In addition, in the numerical ranges described in the disclosure, the upper or lower limit value described in a certain numerical range may be replaced with a value shown in Examples.

In the disclosure, combinations of two or more preferred aspects are more preferred aspects.

In the disclosure, when there are a plurality of substances corresponding to each component, the amount of each component means the total amount of the plurality of substances, unless otherwise specified.

In the disclosure, "(meth)acryl" means acryl and methacryl, and "(meth)acryloyl" means acryloyl and methacryloyl.

### <<Kit for Preparing Curable Composition for Dental Material>>

The kit for preparing a curable composition for a dental material of the disclosure (also simply referred to as the "kit for preparing a curable composition") is a kit for preparing a curable composition for a dental material including a polymerization accelerator, which includes a first preparation including a first monomer and a second preparation including a second monomer, in which at least one of the first preparation or the second preparation contains an organosiloxane including a polymerizable group and a hydroxyl or alkoxy group.

As described above, a silane coupling agent having a polymerizable group may be used for improving the adhesion to ceramic. Meanwhile, in the case of using a silane coupling agent having a polymerizable group, the mechanical properties of the resulting cured product may deteriorate.

In the kit for preparing a curable composition of the disclosure, at least one of the first preparation or the second preparation contains an organosiloxane including a polymerizable group as a silane coupling agent including a polymerizable group and a hydroxyl or alkoxy group. However, it is possible to improve adhesion to ceramics without impairing the mechanical properties of the cured product.

The kit for preparing a curable composition of the disclosure is a kit for preparing a curable composition for a dental material including a polymerization accelerator, in which at least one of a first preparation or a second preparation contains an organosiloxane including a polymerizable group and a hydroxyl or alkoxy group, the first preparation contains a first monomer, and the second preparation contains a second monomer. Thus, the resulting cured product has excellent adhesion to ceramics and is inhibited from decreasing in mechanical properties.

In the disclosure, siloxane refers to a compound having a structure of -Si-O-Si-.

### <Organosiloxane>

At least one of the first preparation or the second preparation contains an organosiloxane including a polymerizable group and a hydroxyl or alkoxy group.

This can improve the adhesion of the cured product to ceramics.

The organosiloxane can be used as a so-called silane coupling agent.

The organosiloxane can be used without any particular limitation as long as it includes a polymerizable group and a hydroxyl or alkoxy group.

It is preferable that the remaining six binding arms of two silicon atoms in one siloxane bond in the organosiloxane of the disclosure, which do not form the siloxane bond, are each independently bonded to an -O-Si group, a monovalent organic group having a polymerizable group, a monovalent organic group not having a polymerizable group, or a hydroxyl group.

It is preferable that in all of the -O-Si groups contained in the organosiloxane, at least one of the four binding arms of Si is bonded to an oxygen atom (-O-) that is bonded to an adjacent Si (i.e., forms a siloxane bond), and the remaining three binding arms are each independently bonded to an -O-Si group, a monovalent organic group having a polymerizable group, a monovalent organic group not having a polymerizable group, or a hydroxyl group. In other words, it is preferable that in a case in which an -O-Si group is bonded to the remaining six binding arms of two silicon atoms in one siloxane bond in an organosiloxane, the remaining three binding arms of the -O-Si group are each independently bonded to an -O-Si group, a monovalent organic group having a polymerizable group, a monovalent organic group not having a polymerizable group, or a hydroxyl group.

In addition, the organosiloxane of the disclosure has a monovalent organic group having at least one polymerizable group and an alkoxy or hydroxyl group, while the monovalent organic group having a polymerizable group and the alkoxy or hydroxyl group are each bonded to the remaining three binding arms of Si in the -O-Si group contained in the organosiloxane, and they may be bonded to Si in the same -O-Si group, or to Si in a different - O-Si group.

The organosiloxane in the disclosure has at least one siloxane bond, but may contain an -O-Si group in addition to the one siloxane bond. For example, in addition to one siloxane bond, it may contain up to 10 -O-Si groups.

In the monovalent organic group having a polymerizable group bonded to Si in the - O-Si group contained in the organosiloxane in the disclosure, examples of the polymerizable group include a (meth)acryloyl group, an epoxy group, and an allyl group.

Among the above, the organosiloxane contains preferably a (meth)acryloyl group as a polymerizable group.

The monovalent organic group having a polymerizable group bonded to Si in the -O-Si group contained in the organosiloxane in the disclosure has preferably from 3 to 30 carbon atoms, more preferably from 4 to 20 carbon atoms, and still more preferably from 5 to 15 carbon atoms.

The monovalent organic group having a polymerizable group bonded to Si in the -O-Si group contained in the organosiloxane in the disclosure may be a simple hydrocarbon group or may contain a urethane bond, an ester bond, an ether bond, or the like.

Examples of the monovalent organic group that does not have a polymerizable group bonded to Si in the -O-Si group contained in the organosiloxane in the disclosure include optionally substituted alkyl and alkoxy groups, with an alkoxy group being preferred.

In the case of the above substitution, examples of the substituent include a hydroxyl group and an amino group.

The monovalent organic group that does not have a polymerizable group bonded to Si in the -O-Si group contained in the organosiloxane in the disclosure has preferably from 1 to 10 carbon atoms, more preferably from 1 to 7 carbon atoms, and still more preferably from 1 to 4 carbon atoms.

The organosiloxane preferably contains a compound represented by the following Formula (X-1).

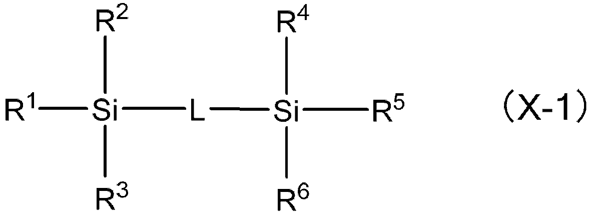

In Formula (X-1), R¹ to R⁶ each independently represent a monovalent organic group having a polymerizable group, a monovalent organic group not having a polymerizable group, or a hydroxyl group, and
L is an oxygen atom or a structure represented by the following Formula (Y-1).

Note that in Formulas (X-1) and (Y-1), at least one of R¹ to R⁸ is a monovalent organic group having a polymerizable group, and at least one of them is an alkoxy or hydroxyl group.

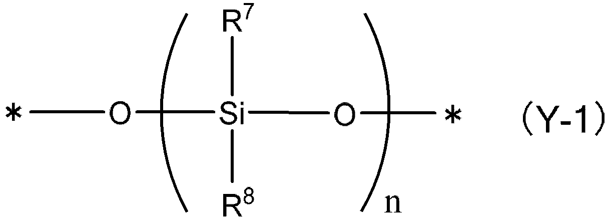

In Formula (Y-1), R⁷ to R⁸ each independently represent a monovalent organic group having a polymerizable group, a monovalent organic group not having a polymerizable group, a hydroxyl group, or an OSi chain, n is an integer of 1 or more, and * represents a bonding site.

The OSi chain is a monovalent organic group having an -O-Si group that forms a siloxane bond with an adjacent Si in Formula (Y-1).

In Formula (X-1), it is preferable that at least one of R¹ to R³ and at least one of R⁴ to R⁶ is a monovalent organic group having a polymerizable group.

In Formula (X-1), it is preferable that at least one of R¹ to R³ and at least one of R⁴ to R⁶ is an alkoxy or hydroxyl group.

In the monovalent organic group having a polymerizable group represented by R¹ to R⁶ in Formula (X-1), examples of the polymerizable group include a (meth)acryloyl group, an epoxy group, and an allyl group.

Among the above, the organosiloxane contains preferably a (meth)acryloyl group as a polymerizable group.

The monovalent organic group having a polymerizable group for R¹ to R⁶ in Formula (X-1) has preferably from 3 to 30 carbon atoms, more preferably from 4 to 20 carbon atoms, and still more preferably from 5 to 15 carbon atoms.

The monovalent organic group having a polymerizable group for R¹ to R⁶ in Formula (X-1) may be a simple hydrocarbon group, but may also contain a urethane bond, an ester bond, an ether bond, or the like.

The monovalent organic group not having a polymerizable group for R¹ to R⁶ in Formula (X-1) includes an optionally substituted alkyl or alkoxy group, with an alkoxy group being preferred.

In the case of the above substitution, examples of the substituent include a hydroxyl group and an amino group.

The monovalent organic group not having a polymerizable group for R¹ to R⁶ in Formula (X-1) has preferably from 1 to 10 carbon atoms, more preferably from 1 to 7 carbon atoms, and still more preferably from 1 to 4 carbon atoms.

Specific aspects, preferred aspects, and the like of the monovalent organic group having a polymerizable group for R⁷ to R⁸ in Formula (Y-1) are the same as the specific aspects, preferred aspects, and the like of the monovalent organic group having a polymerizable group in Formula (X-1).

Specific aspects, preferred aspects, and the like of the monovalent organic group not having a polymerizable group for R⁷ to R⁸ in Formula (Y-1) are the same as the specific aspects, preferred aspects, and the like of the monovalent organic group not having a polymerizable group in Formula (X-1).

In Formula (Y-1), n is preferably an integer from 1 to 8, more preferably an integer from 1 to 6, and still more preferably an integer from 1 to 3.

In Formula (Y-1), OSi is a monovalent organic group having an -O-Si group (hereinafter also referred to as "an -O-Si group bonded to Si in Formula (Y-1)") that forms a siloxane bond (i.e., -Si-O-Si-) with an adjacent Si in Formula (Y-1).

The OSi chain may contain an -O-Si group other than the -O-Si group bonded to Si in Formula (Y-1). In other words, the OSi chain has a chain structure containing one or more -O-Si groups.

The chain structure of the OSi chain contains one or more, preferably from 1 to 10, more preferably from 1 to 5, and still more preferably from 1 to 3 -O-Si groups.

The OSi chain may include a structure in which siloxane bonds (i.e., -Si-O-Si-) are repeated consecutively.

An example of a case in which the chain structure is linear is a case in which the siloxane structure is linear. Similarly, examples of a case in which the chain structure is a branched chain include a case in which the siloxane structure is a branched chain that is branched midway.

It is preferable that in the OSi chain, one of the four binding arms of Si in the -O-Si group that is bonded to Si in Formula (Y-1) is bonded to an oxygen atom (-O-) that is bonded to Si in Formula (Y-1), and the remaining three binding arms are each independently bonded to an -O-Si group (i.e., a group that forms a siloxane bond), a monovalent organic group having a polymerizable group, a monovalent organic group not having a polymerizable group, or a hydroxyl group.

It is preferable that in all of the -O-Si groups contained in the OSi chain (including the -O-Si groups bonded to Si in Formula (Y-1)), at least one of the four binding arms of Si is bonded to an oxygen atom (-O-) bonded to an adjacent Si, and the remaining three binding arms are each independently bonded to an -O-Si group (i.e., a group that forms a siloxane bond), a monovalent organic group having a polymerizable group, a monovalent organic group not having a polymerizable group, or a hydroxyl group. In other words, it is preferable that for example, in a case in which an -O-Si group (i.e., a group that forms a siloxane bond) is bonded to the four binding arms of Si in the -O-Si group bonded to Si in Formula (Y-1), at least one of the four binding arms of Si is bonded to an oxygen atom (-O-) that is bonded to an adjacent Si also in the -O-Si group, and the remaining three binding arms are each independently bonded to an -O-Si group (i.e., a group that forms a siloxane bond), a monovalent organic group having a polymerizable group, a monovalent organic group not having a polymerizable group, or a hydroxyl group.

For all -O-Si groups contained in the OSi chain (including -O-Si groups bonded to Si in Formula (Y-1)), specific aspects, preferred aspects, and the like of the monovalent organic group having a polymerizable group bonded to Si and the monovalent organic group not having a polymerizable group are the same as the specific aspects, preferred aspects, and the like of the monovalent organic group having a polymerizable group and the monovalent organic group not having a polymerizable group in Formula (X-1).

The organosiloxane has a molecular weight of preferably 200 or more, more preferably 250 or more, and still more preferably 300 or more.

The organosiloxane has a molecular weight of preferably 3000 or less, more preferably 2000 or less, still more preferably 1500 or less, and particularly preferably 1000 or less.

The organosiloxane may have a molecular weight of from 200 to 1500.

The total content of an organosiloxane contained in the first preparation and the second preparation is preferably from 0.1% by mass to 15% by mass with respect to the total mass of a curable composition for a dental material to be prepared.

As long as the total organosiloxane content is 0.1% by mass or more with respect to the total mass of a curable composition to be prepared, adhesion to ceramics becomes excellent.

From the above-described viewpoint, the total organosiloxane content is more preferably 1.0% by mass or more and still more preferably 1.5% by mass or more with respect to the total mass of a curable composition to be prepared.

As long as the total organosiloxane content is 15.0 mass% or less with respect to the total mass of a curable composition to be prepared, deterioration of mechanical properties can be further suppressed.

From the above-described viewpoint, the total organosiloxane content is more preferably 12.0% by mass or less, still more preferably 9.0% by mass or less, and particularly preferably 6.0% by mass or less with respect to the total mass of a curable composition to be prepared.

Examples of the method of producing an organosiloxane in the disclosure include a method using an organosilane.

Specific examples of organosilanes are preferably those containing 3-acryloxypropylmethoxysilane or 3-methacryloxypropylmethoxysilane from the viewpoint of the resulting organosiloxane being copolymerized with the polymerizable monomer so as to be cured, and from the viewpoint of improving adhesion to ceramic materials.

The polymerization by hydrolysis and condensation of an organosilane may be carried out by a known method.

For example, the reaction is generally carried out without a solvent, but may be carried out in the presence of an organic solvent that dissolves an organosilane used in the reaction.

The polymerization by hydrolysis and condensation may be carried out by adding water and an acid or base that serves as a catalyst for the hydrolysis reaction to a liquid of the above-described organosilane (including a mixed liquid of a plurality of organosilanes) or a solution thereof.

The synthesis of an organosiloxane in the present disclosure may be carried out by setting appropriate conditions depending on the desired organosiloxane. For example, 0.01 to 0.2 moles of hydrochloric acid are added dropwise to 1 mole of an organosilane at room temperature while stirring, the mixture is heated to about from 50°C to 80°C, and hydrolysis and condensation are carried out for a predetermined time (for example, about from 2 to 72 hours). This is heated to from 60°C to 120°C, thereby distilling off the by-product alcohol under normal pressure. The concentrate is then filtered, thereby obtaining an organosiloxane.

The degree of polymerization may be adjusted by, for example, the heating temperature, the heating time, the amount of the catalyst for hydrolysis and condensation (acid or base), and the like. The resulting organosiloxane can be confirmed by molecular weight analysis by LC/MS (liquid chromatography/mass spectrometry), gel filtration chromatography, or the like.

To synthesize an organosiloxane, literature such as WO 2021/095367 may be referred to.

A commercially available product may be used as the organosiloxane in the disclosure. Examples thereof include: compounds containing epoxy groups such as KR-517 (manufactured by Shin-Etsu Chemical Co., Ltd.), X-24-9590 (manufactured by Shin-Etsu Chemical Co., Ltd.), and KR-516 (manufactured by Shin-Etsu Chemical Co., Ltd.); compounds containing vinyl groups such as KR-511 (manufactured by Shin-Etsu Chemical Co., Ltd.); compounds containing methacryl groups such as KR-513 (manufactured by Shin-Etsu Chemical Co., Ltd.); and compounds containing acrylic groups such as X-40-9296 (manufactured by Shin-Etsu Chemical Co., Ltd.).

### <Polymerization Accelerator>

The kit for preparing a curable composition for a dental material of the disclosure includes a polymerization accelerator.

At least one of the first preparation or the second preparation may include a polymerization accelerator.

The polymerization accelerator can be used without any particular limitation.

The polymerization accelerator contains preferably at least one of an oxidant or a reducing agent, and more preferably an oxidant and a reducing agent.

### (Oxidant)

Examples of the oxidant include an organic peroxide.

### (Organic Peroxide)

A known organic peroxide can be used without any particular limitation. Typical examples of organic peroxides include hydroperoxides, peroxyesters, ketone peroxides, peroxyketals, dialkyl peroxides, diacyl peroxides, and peroxydicarbonates. Among these, hydroperoxides are preferable because the change in operable time is small even when the curable composition is provided in a package and stored for a long period of time. The organic peroxide may be used singly, or in combination of two or more kinds thereof.

More specific examples of hydroperoxides include Hydroperoxides include cumene hydroperoxide, t-butyl hydroperoxide, t-hexyl hydroperoxide, p-menthane hydroperoxide, diisopropylbenzene hydroperoxide, 1,1,3,3-tetramethylbutyl hydroperoxide, and t-amyl hydroperoxide.

Any peroxyester can be used without any limitation as long as it contains an acyl group on one side of a peroxy group (-OO- group) and a hydrocarbon group (or a group similar thereto) on the other side. Specific examples thereof include α,α-bis(neodecanoylperoxy)diisopropylbenzene, cumylperoxy neodecanoate, 1,1,3,3-tetramethylbutylperoxy neodecanoate, 1-cyclohexyl-1-methylethylperoxy neodecanoate, t-hexyl peroxyneodecanoate, t-butyl peroxyneodecanoate, t-hexyl peroxypivalate, t-butyl peroxypivalate, 1,1,3,3-tetramethylbutylperoxy-2-ethylhexanoate, 2,5-dimethyl-2,5-bis(2-ethylhexanoylperoxy)hexane, 1-cyclohexyl-1-methylethylperoxy-2-ethylhexanoate, t-hexylperoxy-2-ethylhexanoate, t-butylperoxy-2-ethylhexanoate, t-butylperoxy isobutyrate, t-hexylperoxy isopropyl monocarbonate, t-butylperoxy maleic acid, t-butylperoxy 3,5,5-trimethylhexanoate, t-butylperoxy laurate, 2,5-dimethyl-2,5-bis(m-toluoylperoxy)hexane, t-butylperoxy isopropyl monocarbonate, t-butylperoxy-2-ethylhexyl monocarbonate, t-hexylperoxy benzoate, 2,5-dimethyl-2,5-bis(benzoylperoxy)hexane, t-butylperoxy acetate, t-butylperoxy-m-toluoylbenzoate, t-butylperoxy benzoate, and bis(t-butylperoxy)isophthalate. These can be used singly, or in combination of two or more kinds thereof, as appropriate.

Examples of ketone peroxides include methyl ethyl ketone peroxide, cyclohexanoperoxide, methylcyclohexanone peroxide, methylacetoacetate peroxide, and acetylacetone peroxide.

Examples of peroxyketals include 1,1-bis(t-hexylperoxy)3,3,5-trimethylcyclohexane, 1,1-bis(t-hexylperoxy)cyclohexane, 1,1-bis(t-butylperoxy)3,3,5-trimethylcyclohexanone, 1,1-bis(t-butylperoxy)cyclohexane, 1,1-bis(t-butylperoxy)cyclodecane, 2,2-bis(t-butylperoxy)butane, n-butyl 4,4-bis(t-butylperoxy)valerate, and 2,2-bis(4,4-di-t-butylperoxycyclohexyl)propane.

Examples of dialkyl peroxides include α,α-bis(t-butylperoxy)diisopropylbenzene, dicumyl peroxide, 2,5-dimethyl-2,5-bis(t-butylperoxy)hexane, t-butylcumyl peroxide, di-t-butyl peroxide, and 2,5-dimethyl-2,5-bis(t-butylperoxy)hexyne-3.

Examples of diacyl peroxides include isobutyryl peroxide, 2,4-dichlorobenzoyl peroxide, 3,5,5-trimethylhexanoyl peroxide, octanoyl peroxide, lauroyl peroxide, stearyl peroxide, succinic acid peroxide, m-toluoylbenzoyl peroxide, and benzoyl peroxide.

Examples of peroxydicarbonates include di-n-propyl peroxydicarbonate, diisopropyl peroxydicarbonate, bis(4-t-butylcyclohexyl) peroxydicarbonate, di-2-ethoxyethyl peroxydicarbonate, di-2-ethylhexyl peroxydicarbonate, di-2-methoxybutyl peroxydicarbonate, and di(3-methyl-3-methoxybutyl) peroxydicarbonate.

The content of the organic peroxide is preferably from 0.01 parts by mass to 5 parts by mass, more preferably from 0.05 parts by mass to 2 parts by mass, and still more preferably from 0.05 parts by mass to 1 part by mass with respect to 100 parts by mass in total of the first preparation and the second preparation.

### (Reducing Agent)

The reducing agent may contain a reducing agent (A) as a transition metal compound and a reducing agent (B) other than the transition metal compound.

### (Transition Metal Compound)

The reducing agent (A) is a transition metal compound.

The transition metal compound is preferably a compound that is soluble in monomer components described later in the kit for preparing a curable composition.

Examples of the transition metal compound include copper compounds, vanadium compounds, molybdenum compounds, scandium compounds, titanium compounds, chromium compounds, manganese compounds, iron compounds, cobalt compounds, and nickel compounds.

Among the above, the transition metal compound contains preferably at least one of a copper compound or a vanadium compound, and more preferably a copper compound.

Examples of copper compounds include copper carboxylates such as copper acetate, copper isobutyrate, copper gluconate, copper citrate, copper phthalate, copper tartrate, copper oleate, copper octylate, copper octenoate, copper naphthenate, copper methacrylate, and copper 4-cyclohexylbutyrate; copper β-diketones such as copper acetylacetone, copper trifluoroacetyl acetone, copper hexafluoroacetylacetone, copper 2,2,6,6-tetramethyl-3,5-heptanedionato, and copper benzoylacetone; copper β-ketoesters such as copper ethyl acetoacetate; copper alkoxides such as copper methoxide, copper ethoxide, copper isopropoxide, copper 2-(2-butoxyethoxy)ethoxide, and copper 2-(2-methoxyethoxy)ethoxide; copper dithiocarbamates such as copper dimethyldithiocarbamate; salts of copper with inorganic acids such as copper nitrate; and copper chloride.

These can be used singly, or in combination of two or more kinds thereof, as appropriate.

Among these, from the viewpoints of solubility and reactivity in monomers, copper carboxylates, copper β-diketones, and copper β-ketoesters are preferable, and copper acetate and copper acetylacetone are more preferable.

Examples of vanadium compounds include vanadyl acetylacetonate, vanadium naphthenate (III), vanadyl stearate, vanadium benzoylacetonate, bis(maltolato)oxovanadium (IV), and oxobis(1-phenyl-1,3-butanedionate)vanadium (IV).

For the kit for preparing a curable composition for a dental material of the disclosure, the content of the transition metal compound is preferably from 0.0005 parts by mass to 3.0 parts by mass, more preferably from 0.001 parts by mass to 2.0 parts by mass, and still more preferably 0.01 parts by mass to 1.0 parts by mass with respect to 100 parts by mass in total of the first preparation and the second preparation.

Examples of the reducing agent (B) other than the transition metal compound include thioureas, ascorbic acids, sulfinic acids, and amine compounds.

It is preferable that the reducing agent (B) contains at least one selected from the group consisting of a thiourea, an ascorbic acid, a sulfinic acid, and an amine compound.

### (Thioureas)

Thioureas refer to thioureas and thiourea derivatives.

Examples of thioureas include acetylthiourea, phenylthiourea, triethylthiourea, tetramethylthiourea, dimethylthiourea, and diphenylthiourea.

### (Ascorbic Acids)

In the disclosure, ascorbic acids refer to ascorbic acid, ascorbic acid derivatives, or salts thereof.

It is preferable that an ascorbic acid derivative or a salt thereof in the disclosure has, for example, a structure in which at least one of four hydroxyl groups contained in ascorbic acid is replaced with an ether bond bonded to a carbon atom (hereinafter also referred to as an "ether derivative of ascorbic acid"), or a structure in which at least one of four hydroxyl groups contained in ascorbic acid is replaced with an ester bond bonded to a carbon atom (hereinafter also referred to as an "ester derivative of ascorbic acid").

Examples of salts of ascorbic acids in the disclosure include alkali metal salts of ascorbic acid or an ascorbic acid derivative, and alkaline earth metal salts of ascorbic acid or an ascorbic acid derivative.

More specific examples thereof include sodium salts, potassium salts, magnesium salts, calcium salts, and the like of ascorbic acids. Of these, sodium salts and calcium salts are preferable from the viewpoint of the balance between polymerizability and adhesiveness.

Examples of ester derivatives of ascorbic acid include those having a structure formed by a reaction of at least one of the four hydroxyl groups contained in ascorbic acid with carboxylic acid (e.g., a carboxylic acid having from 2 to 30 carbon atoms).

Suitable examples of carboxylic acids having from 2 to 30 carbon atoms include fatty acids such as caprylic acid, capric acid, lauric acid, myristic acid, palmitic acid, stearic acid, arachidic acid, behenic acid, lignoceric acid, cerotic acid, myristoleic acid, palmitoleic acid, sapienic acid, oleic acid, elaidic acid, vaccenic acid, linoleic acid, linoleelaidic acid, α-linolenic acid, arachidonic acid, eicosapentaenoic acid, erucic acid, and docosahexaenoic acid.

Examples of an ether derivative of ascorbic acid include those having a structure formed by a reaction of at least one of the four hydroxyl groups contained in ascorbic acid with an alkyl halide.

Examples of the alkyl halide include alkyl chlorides having from 1 to 30 carbon atoms and alkyl bromides having from 1 to 30 carbon atoms.

Another example of an ether derivative of ascorbic acid is one having a structure in which at least two of the four hydroxyl groups contained in ascorbic acid form an ether bond with the same carbon atom (i.e., an acetal structure).

An example of the ether derivative of ascorbic acid containing an acetal structure in the disclosure is a compound represented by the following Formula (B).

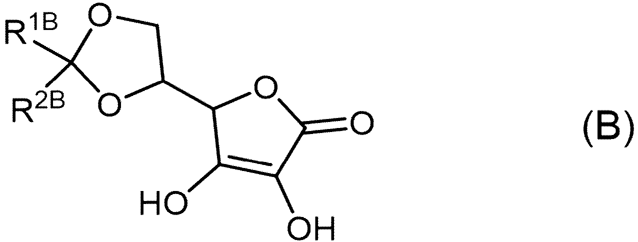

In Formula (B), R^{1B} and R^{2B} each independently represent a hydrogen atom or a monovalent organic group.

At least one of R^{1B} or R^{2B} is preferably a monovalent organic group.

The monovalent organic group for R^{1B} and R^{2B} is preferably an organic group having from 1 to 12 carbon atoms, more preferably an organic group having from 1 to 10 carbon atoms, and still more preferably an organic group having from 3 to 10 carbon atoms.

In Formula (B), one of R^{1B} or R^{2B} may be a hydrogen atom, and the other of R^{1B} or R^{2B} may be a monovalent organic group having from 1 to 10 carbon atoms.

The monovalent organic group for R^{1B} and R^{2B} may be an organic group containing an oxygen atom, a nitrogen atom, a sulfur atom, or the like, or may be a hydrocarbon group to which an oxygen atom, a nitrogen atom, a sulfur atom, or the like is bonded, or may be a hydrocarbon group.

The salt of the ascorbic acid derivative in the disclosure may be a salt of a compound represented by Formula (B) or an alkali metal salt or alkaline earth metal salt of a compound represented by Formula (B). From the viewpoint of the balance between polymerizability and adhesiveness, a calcium salt of a compound represented by Formula (B) is preferable.

In a case in which the salt of the ascorbic acid derivative in the disclosure is a salt of a compound represented by Formula (B), it is preferable that R^{1B} and R^{2B} each independently represent a hydrogen atom or a monovalent organic group. It is more preferable that one of R^{1B} or R^{2B} is a hydrogen atom, and the other of R^{1B} or R^{2B} is a monovalent organic group having from 1 to 10 carbon atoms, or R^{1B} and R^{2B} are each independently a monovalent organic group having from 1 to 10 carbon atoms.

In a case in which one of R^{1B} or R^{2B} is a hydrogen atom, and the other of R^{1B} or R^{2B} is a monovalent organic group having from 1 to 10 carbon atoms, the other of R^{1B} or R^{2B} is preferably a monovalent organic group having from 1 to 5 carbon atoms, more preferably a monovalent organic group having from 1 to 3 carbon atoms, still more preferably a methyl, ethyl, propyl, or isopropyl group, and particularly preferably an isopropyl group.

In a case in which R^{1B} and R^{2B} are each independently a monovalent organic group having from 1 to 10 carbon atoms, R^{1B} and R^{2B} are each independently preferably a monovalent organic group having from 1 to 5 carbon atoms, more preferably a monovalent organic group having from 1 to 3 carbon atoms, and still more preferably a methyl, ethyl, propyl, or isopropyl group, and both of them are particularly preferably methyl groups.

Compounds represented by Formula (B) may be synthesized, for example, by using known acetalization reactions.

### (Sulfinic Acids)

In the disclosure, sulfinic acids refer to sulfinic acid, sulfinic acid derivatives, and salts thereof.

Examples of aromatic sulfinic acids include lithium salts of benzene sulfinic acid, p-toluenesulfinic acid, o-toluenesulfinic acid, ethylbenzenesulfinic acid, decylbenzenesulfinic acid, dodecylbenzenesulfinic acid, 2,4,6-trimethylbenzenesulfinic acid, 2,4,6-triisopropylbenzenesulfinic acid, chlorobenzenesulfinic acid, naphthalenesulfinic acid, and the like, sodium salts, potassium salts, rubidium salts, cesium salts, magnesium salts, calcium salts, strontium salts, iron salts, zinc salts, ammonium salts, tetramethylammonium salts, and tetraethylammonium salts.

### (Amine Compounds)

Examples of amine compounds include aromatic amines, aliphatic amines, and heterocyclic amines.

### (Aromatic Amines)

Known aromatic secondary amines, aromatic tertiary amines, and the like may be used as aromatic amines.

Examples of aromatic secondary amines and aromatic tertiary amines include N,N-bis(2-hydroxyethyl)-3,5-dimethylaniline, diethanol-p-toluidine (DEPT), N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-diisopropylaniline, N,N-bis(2-hydroxyethyl)-3,5-di-t-butylaniline, N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, and N,N-dimethyl-3,5-di-t-butylaniline.

In the disclosure, aromatic amines refer to aromatic amines that do not fall under the category of heterocyclic amines described later.

### (Aliphatic Amines)

Examples of aliphatic amines include primary aliphatic amines such as n-butylamine, n-hexylamine, and n-octylamine; secondary aliphatic amines such as diisopropylamine, dibutylamine, and N-methylethanolamine;
and tertiary aliphatic amines such as N-methyldiethanolamine, N-ethyldiethanolamine, N-n-butyldiethanolamine, N-lauryldiethanolamine, 2-(dimethylamino)ethyl(meth)acrylate, N-methyldiethanolamine di(meth)acrylate, N-ethyldiethanolamine di(meth)acrylate, triethanolamine tri(meth)acrylate, triethanolamine, trimethylamine, triethylamine, and tributylamine.

### (Heterocyclic Amines)

Heterocyclic amines are amines that contain a heterocyclic ring.

Examples of heterocyclic amines include heterocyclic amines that can function as monodentate, bidentate, or tridentate heterocyclic electron donor ligands.

Specific examples thereof include: heterocyclic amines derived from unsubstituted or substituted heteroarenes such as furan, thiophene, pyrrole, pyridine, bipyridine, picolinimine, γ-pyran, γ-thiopyran, phenanthroline, pyrimidine, bis-pyrimidine, pyrazine, indole, coumarin, thionaphthene, carbazole, dibenzofuran, dibenzothiophene, pyrazole, imidazole, benzimidazole, oxazole, thiazole, bis-thiazole, isoxazole, isothiazole, quinoline, biquinoline, isoquinoline, biisoquinoline, acridine, chroman, phenazine, phenoxazine, phenothiazine, triazine, thianthrene, purine, bismidazole, and bisoxazoline; or salts thereof.

Preferred heterocyclic amines include 1H-benzotriazole (BTA), 5-methyl-1H-benzotriazole, and benzimidazole.

For the kit for preparing a curable composition for a dental material of the disclosure, it is preferable that the oxidant contains an organic peroxide, the reducing agent contains a reducing agent (A) as a transition metal compound and a reducing agent (B) other than the transition metal compound, the first preparation contains an organic peroxide and the reducing agent (A), and the second preparation contains the reducing agent (B).

At least one of the first preparation or the second preparation each independently contains a transition metal compound and an organic peroxide.

The expression "at least one of the first preparation or the second preparation each independently contains a transition metal compound and an organic peroxide" means that at least one of the first preparation or the second preparation contains a transition metal compound and at least one of the first preparation or the second preparation contains an organic peroxide.

In a case in which a transition metal compound is contained in both of the first preparation and the second preparation, a transition metal compound contained in the first preparation and a transition metal compound contained in the second preparation may be the same or different.

This also applies to organic peroxides.

### <Monomers>

In the kit for preparing a curable composition of the disclosure, the first preparation contains a first monomer, and the second preparation contains a second monomer.

The first monomer and the second monomer may be the same monomer or different monomers.

Known monomers can be used as the first monomer and the second monomer.

The first monomer and the second monomer may be a monomer not containing an acidic group or a monomer containing an acidic group (hereinafter also referred to as "acidic monomer").

It is preferable that the first monomer and the second monomer contains a monomer not containing an acidic group.

The monomer is a monomer that undergoes a radical polymerization reaction due to the action of the ascorbic acid, transition metal compound, organic peroxide, or the like of the disclosure so as to become a polymer.

Monomers constituting the monomer in the disclosure are not limited to one kind, and may be two or more kinds. Examples of a monomer not containing an acidic group include a (meth)acrylate monomer not containing an acidic group. Examples of the (meth)acrylate monomer not containing an acidic group include monofunctional monomers, bifunctional monomers, and tri- or higher functional monomers.

The content of monomers in the disclosure (i.e., the total amount of the first monomer and the second monomer in a curable composition to be prepared) is preferably from 10% by mass to 90% by mass, more preferably from 20% by mass to 75% by mass, and still more preferably from 30% by mass to 60% by mass with respect to the total mass of a curable composition to be prepared.

Examples of monofunctional monomers include 2-hydroxyethyl (meth)acrylate, 3-hydroxypropyl (meth)acrylate, 2-hydroxypropyl (meth)acrylate, 1,3-dihydroxypropyl (meth)acrylate, and 2,3-dihydroxypropyl (meth)acrylate. Of these, 2-hydroxyethyl methacrylate (HEMA) is preferable.

Examples of aromatic compound-based bifunctional monomers include 2,2-bis((meth)acryloyloxyphenyl)propane, 2,2-bis[4-(3-(meth)acryloyloxy)-2-hydroxypropoxyphenyl]propane, 2,2-bis(4-(meth)acryloyloxyethoxyphenyl)propane, 2,2-bis(4-(meth)acryloyloxypolyethoxyphenyl)propane, and 2,2-bis(4-(meth)acryloyloxypolypropoxyphenyl)propane.

Of these, 2,2-bis[4-(3-(methacryloyloxy)-2-hydroxypropoxyphenyl]propane (commonly known as "Bis-GMA") and 2,2-bis(4-methacryloyloxypolyethoxyphenyl)propane are preferable.

Examples of aliphatic compound-based bifunctional monomers include erythritol di(meth)acrylate, sorbitol di(meth)acrylate, mannitol di(meth)acrylate, pentaerythritol di(meth)acrylate, dipentaerythritol di(meth)acrylate, glycerol di(meth)acrylate, ethylene glycol di(meth)acrylate, diethylene glycol di(meth)acrylate, triethylene glycol di(meth)acrylate, propylene glycol di(meth)acrylate, butylene glycol di(meth)acrylate, acrylate, neopentyl glycol di(meth)acrylate, polyethylene glycol di(meth)acrylate, 1,3-butanediol di(meth)acrylate, 1,5-pentanediol di(meth)acrylate, 1,6-hexanediol di(meth)acrylate, 1,10-decanediol di(meth)acrylate, 2,2,4-trimethylhexamethylenebis(2-carbamoyloxyethyl)dimethacrylate (UDMA), and 1,2-bis(3-methacryloyloxy-2-hydroxypropyloxy)ethane.

Of these, glycerol dimethacrylate, triethylene glycol dimethacrylate (TEGDMA), 1,6-hexanediol dimethacrylate (HexDMA), neopentyl glycol dimethacrylate (NPG), 2,2,4-trimethylhexamethylenebis(2-carbamoyloxyethyl)dimethacrylate (UDMA) and 1,2-bis(3-methacryloyloxy-2-hydroxypropyloxy)ethane are preferable.

Examples of tri- or higher functional monomers include trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol tri(meth)acrylate, dipentaerythritol tetra(meth)acrylate, dipentaerythritol penta(meth)acrylate, N,N-(2,2,4-trimethylhexamethylene)bis[2-(aminocarboxy)propane-1,3-diol]tetramethacrylate, and 1,7-diacryloyloxy-2,2,6,6-tetraacryloyloxymethyl-4-oxyheptane.

The above-described monomers may be mixed singly, or in combination of two or more kinds thereof.

The content of the monomer not containing an acidic group is preferably in a range of from 10 parts by mass to 100 parts by mass, more preferably in a range of from 20 parts by mass to 100 parts by mass, and still more preferably in a range of from 50 parts by mass to 100 parts by mass with respect to 100 parts by mass in total of monomer components in the kit for preparing a curable composition of the disclosure.

In a case in which the monomer components in the kit for preparing a curable composition of the disclosure include acidic monomers described later, the content of the monomer not containing an acidic group is preferably from 10 parts by mass to 99 parts by mass, more preferably from 30 parts by mass to 97 parts by mass, and still more preferably from 50 parts by mass to 95 parts by mass with respect to 100 parts by mass in total of monomer components in the kit for preparing a curable composition of the disclosure.

It is preferable that the first monomer and the second monomer contain a (meth)acrylic monomer (C) having a molecular weight of from 100 to 5000.

The molecular weight of a (meth)acrylic monomer (C) is more preferably from 120 to 3000, still more preferably from 150 to 2000, and particularly preferably from 200 to 1000.

The content of the (meth)acrylic monomer (C) with respect to the total content of the first monomer and the second monomer is preferably 50% by mass or more, more preferably 70% by mass or more, and still more preferably 90% by mass or more.

### <Acidic Monomer>

For the kit for preparing a curable composition of the disclosure, it is preferable that either one of the first monomer or the second monomer contains an acidic monomer, and it is more preferable that the first monomer contains an acidic monomer.

More specifically, it is preferable that either the first monomer in the first preparation or the second monomer in the second preparation contains an acidic monomer in the kit for preparing a curable composition of the disclosure.

Since at least one of the first preparation or the second preparation contains an acidic monomer, for example, in a case in which, the kit for preparing a curable composition of the disclosure is used for dental application, favorable tooth substance and a high level of adhesion to dental prosthetic materials can be imparted.

Examples of the acidic monomer include monomers having at least one acidic group, such as a phosphoric acid group, a pyrophosphoric acid group, a thiophosphoric acid group, a phosphonic acid group, a sulfonic acid group, or a carboxylic acid group, and at least one polymerizable group, such as an acryloyl group, a methacryloyl group, a vinyl group, or a styrene group.

The acidic monomer has an affinity for the adherend and has a demineralizing effect on tooth substance.

Examples of a phosphoric acid group-containing monomer include (meth)acryloyloxyalkyl dihydrogen phosphates such as 10-(meth)acryloyloxydecyl dihydrogen phosphate (MDP), bis[2-(meth)acryloyloxyethyl]hydrogen phosphate, bis[4-(meth)acryloyloxybutyl]hydrogen phosphate, bis[6-(meth)acryloyloxyhexy]hydrogen phosphate, bis[8-(meth)acryloyloxyoctyl]hydrogen phosphate, bis[9-(meth)acryloyloxynonyl]hydrogen phosphate, bis[10-(meth)acryloyloxydecyl]hydrogen phosphate, 1,3-di(meth)acryloyloxypropyl dihydrogen phosphate, 2-(meth)acryloyloxyethyl phenyl hydrogen phosphate, 2-(meth)acryloyloxyethyl-2-bromoethyl hydrogen phosphate, bis[2-(meth)acryloyloxy-(1-hydroxymethyl)ethyl]hydrogen phosphate, and acid chlorides, alkali metal salts, and ammonium salts thereof.

Examples of a pyrophosphoric acid group-containing monomer include bis[2-(meth)acryloyloxyethyl]pyrophosphate, bis[4-(meth)acryloyloxybutyl]pyrophosphate, and acid chlorides, alkali metal salts, and ammonium salts thereof.

Examples of a thiophosphoric acid group-containing monomer include 2-(meth)acryloyloxyethyl dihydrogen thiophosphate, 3-(meth)acryloyloxypropyl dihydrogen thiophosphate, and acid chlorides, alkali metal salts, and ammonium salts thereof.

Examples of a phosphonic acid group-containing monomer include 2-(meth)acryloyloxyethyl phenyl phosphonate, 5-(meth)acryloyloxypentyl-3-phosphonopropionate, and acid chlorides, alkali metal salts and ammonium salts thereof.

Examples of a sulfonic acid group-containing monomer include 2-(meth)acrylamide-2-methylpropanesulfonic acid, styrenesulfonic acid, and 2-sulfoethyl(meth)acrylate.

Examples of a carboxylic acid group-containing monomer include a monomer containing one carboxy group in the molecule and a monomer containing a plurality of carboxy groups in the molecule.

Examples of a monomer containing one carboxy group in the molecule include (meth)acrylic acid, N-(meth)acryloylglycine, N-(meth)acryloylaspartic acid, O-(meth)acryloyltyrosine, N-(meth)acryloyltyrosine, N-(meth)acryloylphenylalanine, N-(meth)acryloyl-p-aminobenzoic acid, N-(meth)acryloyl-o-aminobenzoic acid, p-vinylbenzoic acid, 2-(meth)acryloyloxybenzoic acid, 3-(meth)acryloyl Acryloyloxybenzoic acid, 4-(meth)acryloyloxybenzoic acid, N-(meth)acryloyl-5-aminosalicylic acid, N-(meth)acryloyl-4-aminosalicylic acid, 2-(meth)acryloyloxyethyl hydrogen succinate, 2-(meth)acryloyloxyethyl hydrogen phthalate, 2-(meth)acryloyloxyethyl hydrogen maleate, and acid halides thereof.

Examples of a monomer containing a plurality of carboxy groups in the molecule include 6-(meth)acryloyloxyhexane-1,1-dicarboxylic acid, 9-(meth)acryloyloxynonane-1,1-dicarboxylic acid, 10-(meth)acryloyloxydecane-1,1-dicarboxylic acid, 11-(meth)acryloyloxyundecane-1,1-dicarboxylic acid, 12-(meth)acryloyloxydodecane-1,1-dicarboxylic acid, 13-(meth)acryloyloxytridecane-1,1-dicarboxylic acid, 4-(meth)acryloyloxyethyl trimellitate, 4-(meth)acryloyloxyethyl trimellitate anhydride, 4-(meth)acryloyloxybutyl trimellitate, 4-(meth)acryloyloxyhexyl trimellitate, 4-(meth)acryloyloxydecyl trimellitate, 2-(meth)acryloyloxyethyl-3'-(meth)acryloyloxy-2'-(3,4-dicarboxybenzoyloxy)propyl succinate, dihydroxyethyl methacrylate trimethylhexyl dicarbamate, and acid anhydrides or acid halides thereof.

Among the above-described acidic monomers, in view of high adhesive strength to the adherend, 10-(meth)acryloyloxydecyl dihydrogen phosphate (MDP), 1,3-di(meth)acryloyloxypropyl dihydrogen phosphate, 2-(meth)acryloyloxyethyl dihydrogen phosphate, 4-(meth)acryloyloxyethyl trimellitate anhydride, 4-(meth)acryloyloxyethyl trimellitate, 2-(meth)acrylamido-2-methylpropanesulfonic acid, 11-(meth)acryloyloxyundecane-1,1-dicarboxylic acid, and dihydroxyethyl methacrylate trimethylhexyl dicarbamate are preferable.

The above-described acidic monomer may be used singly, or in combination of two or more kinds thereof.

The content of the acidic monomer is preferably from 1 part by mass to 50 parts by mass, more preferably from 3 parts by mass to 40 parts by mass, and still more preferably from 5 parts by mass to 30 parts by mass with respect to 100 parts by mass as the total amount of monomer components in the kit for preparing a curable composition of the disclosure.

When the content of the acidic monomer is 1 part by mass or more, it is easy to obtain a high level of adhesion to various adherends.

In addition, when the content of the acidic monomer is 50 parts by mass or less, the balance between polymerizability and adhesiveness can be easily maintained. The total amount of monomer components means the total amount of an acidic monomer and the above-described monomer not containing an acidic group.

Regarding the monomers in the disclosure, for example, monomers described in known literature such as WO 2012/157566, WO 2015/015220, WO 2015/015221, and JP-A No. 2016-094482 can be used.

In the kit for preparing a curable composition of the disclosure, at least one of the first preparation or the second preparation may contain at least one compound (1) selected from the group consisting of a phosphonite compound, a phosphite compound, and a sulfite compound from the viewpoint of improving the stability and polymerization of ascorbic acids.

### <Phosphonite Compound>

The phosphonite compound may be any trivalent organic phosphorus compound in which a carbon atom is bonded to a phosphorus atom.

Specific examples of the phosphonite compound contained in the compound (1) in the disclosure include tetrakis(2,4-di-tert-butylphenyl)-4,4'-biphenylene-di-phosphonite, tetrakis(2,4-di-tert-butyl-5-methylphenyl)-4,4'-biphenylene-di-phosphonite, tetrakis(2,4-di-tert-butylphenyl)-4,3'-biphenylene-di-phosphonite, tetrakis(2,4-di-tert-butylphenyl)-3,3'-biphenylene-di-phosphonite, tetrakis(2,6-di-tert-butylphenyl)-4,4'-biphenylene-di-phosphonite, tetrakis(2,6-di-tert-butylphenyl)-4,3 '-biphenylene-di-phosphonite, tetrakis(2,6-di-tert-butylphenyl)-3,3'-biphenylene-di-phosphonite, bis(2,4-di-tert-butylphenyl)-4-phenyl-phenylphosphonite, bis(2,4-di-tert-butylphenyl)-3-phenyl-phenylphosphonite, bis(2,6-di-n-butylphenyl)-3-phenyl-phenylphosphonite, bis(2,6-di-tert-butylphenyl)-4-phenyl-phenylphosphonite, and bis(2,6-di-tert-butylphenyl)-3-phenyl-phenylphosphonite.

Examples of the phosphite compound include triphenyl phosphite, trisnonylphenyl phosphite, tricresyl phosphite, diphenyl mono(2-ethylhexyl) phosphite, diphenyl monodecyl phosphite, diphenyl mono(tridecyl) phosphite, and tris(2,4-di-tert-butylphenyl) phosphite.

Examples of the sulfite compound include ethylene sulfite, propylene sulfite, dimethyl sulfite, diethyl sulfite, ethyl methyl sulfite, methyl-n-propyl sulfite, ethyl-n-propyl sulfite, di-n-propyl sulfite, diphenyl sulfite, methyl phenyl sulfite, ethyl sulfite, dibenzyl sulfite, benzyl methyl sulfite, and benzyl ethyl sulfite.

The total content of the compound (1) contained in the first preparation and the second preparation is preferably from 0.1 parts by mass to 5 parts by mass, and more preferably from 0.2 parts by mass to 2 parts by mass with respect to 100 parts by mass as the total amount of the first preparation and the second preparation from the viewpoint of curability.

At least one of the first preparation or the second preparation may contain a polymerization accelerator (2) in the kit for preparing a curable composition of the disclosure.

The polymerization accelerator (2) in the disclosure is not particularly limited, and, for example, an inorganic salt can be mentioned.

Examples of the inorganic salt include sodium sulfite, calcium sulfite, potassium sulfite, potassium nitrate, potassium chloride, potassium sulfate, and sodium chloride.

The total content of the polymerization accelerator (2) contained in the first preparation and the second preparation is preferably from 0.001 parts by mass to 5.0 parts by mass, more preferably from 0.01 parts by mass to 3.0 parts by mass, and still more preferably from 0.1 parts by mass to 2.0 parts by mass with respect to 100 parts by mass as the total amount of the first preparation and the second preparation from the viewpoint of curability.

The total content of the inorganic salt contained in the first preparation and the second preparation is preferably from 0.001 parts by mass to 5.0 parts by mass, more preferably from 0.01 parts by mass to 3.0 parts by mass, and still more preferably from 0.1 parts by mass to 2.0 parts by mass with respect to 100 parts by mass as the total amount of the first preparation and the second preparation from the viewpoint of curability.

At least one of the first preparation or the second preparation in the disclosure may contain a silanol condensing agent.

The silanol condensing agent can be used without any particular limitation.

Examples of the condensing agent include: titanium-based esters such as titanium(IV) tetrabutoxide, titanium(IV) tetrapropoxide, titanium(IV) bis(ethylacetoacetate)diisopropoxide, and titanium(IV) bis(ethylhexoxy)bis(2-ethyl-3-hydroxyhexoxide); organoaluminum compounds such as aluminum(III) acetylacetonate, aluminum trisethylacetoacetate, and diisopropoxyaluminum ethylacetoacetate; and metallic catalysts such as chelate compounds, including zirconium(IV) tetraacetylacetonate, titanium(IV) acetylacetonate, and titanium(IV) bis(acetylacetonate)diisopropoxide. The silanol condensing agent may be used singly, or in combination of two or more kinds thereof.

### <Filler>

At least one of the first preparation or the second preparation may contain a filler, and the first preparation and the second preparation preferably contain a filler in the kit for preparing a curable composition of the disclosure.

The filler may be mixed singly, or in combination of two or more kinds thereof. Examples of the filler include an inorganic filler, an organic filler, and a composite filler of an inorganic filler and an organic filler.

Examples of the inorganic filler include fine powders of various glasses (mainly composed of silicon dioxide and containing oxides of heavy metals, boron, aluminum, and the like, if necessary), various ceramics, diatomaceous earth, kaolin, clay minerals (such as montmorillonite,), activated clay, synthetic zeolite, mica, calcium fluoride, ytterbium fluoride, calcium carbonate, calcium phosphate, aluminum sulfate, barium sulfate, calcium sulfate, zirconium dioxide, titanium dioxide, aluminum oxide, boron oxide, barium oxide, lanthanum oxide, strontium oxide, zinc oxide, calcium oxide, lithium oxide, sodium oxide, bismuth oxide, yttrium oxide, calcium phosphate, hydroxyapatite, aluminum hydroxide, sodium fluoride, potassium fluoride, sodium monofluorophosphate, lithium fluoride, and ytterbium fluoride.

From the viewpoints of adhesive strength and ease of handling, a fine powder having a primary particle size of from 0.001 µm to 100 µm is preferably used.

Specific examples of such an inorganic filler include barium borosilicate glass powder (such as 8235, GM27884, and G018-053 (manufactured by Shott)), strontium boroaluminosilicate glass powder (such as G018-093 and G018-163 (manufactured by Shott)), lanthanum glass powder (such as GM31684 and G018-161 (manufactured by Shott)), fluoroaluminosilicate glass powder (G018-091 and Schott G018-117 (manufactured by Shott), zirconium and/or cesium-containing boroaluminosilicate glass fine powders (G018-307, G018-308 and G018-310 (manufactured by Shott), silica fine powders (AEROSIL (registered trademark) OX50, AEROSIL 50, AEROSIL 200, AEROSIL 380, AEROSIL R972, AEROSIL 130 (manufactured by Evonik), and the like).

Examples of the organic filler include fine powders of polymethyl methacrylate, polyethyl methacrylate, polyfunctional methacrylate polymers, polyamide, polystyrene, polyvinyl chloride, chloroprene rubber, nitrile rubber, and styrene-butadiene rubber.

Examples of the composite filler of inorganic and organic fillers include those in which inorganic fillers are dispersed in organic fillers, and inorganic/organic composite fillers in which inorganic fillers are coated with various polymers.

In order to improve the curability, mechanical strength, and ease of handling, the filler may be used after being surface-treated with a known surface treatment agent such as a silane coupling agent. Examples of the surface treatment agent include vinyltrimethoxysilane, vinyltriethoxysilane, vinyltrichlorosilane, vinyltri(β-methoxyethoxy)silane, γ-methacryloyloxypropyltrimethoxysilane, γ-glycidoxypropyltrimethoxysilane, γ-mercaptopropyltrimethoxysilane, and γ-aminopropyltriethoxysilane.

The total content of the fillers contained in the first preparation and the second preparation is preferably in a range of from 10% by mass to 80% by mass, more preferably in a range of from 30% by mass to 80% by mass, and still more preferably in a range of from 50% by mass to 75% by mass with respect to the total mass of a curable composition to be prepared.

### (Non-Conductive Filler)

It is preferable that at least one of the first preparation or the second preparation contains a non-conductive filler in the kit for preparing a curable composition of the disclosure.

The non-conductive filler means a filler having a resistance value of 1.00 × 10⁻⁴ Ωm or more.

The upper limit of the resistance value for the non-conductive filler is not particularly limited, and may be, for example, 1.00 × 10²⁰ Ωm.

Examples of materials for the non-conductive filler include: organic substances such as polyethylene, polystyrene, phenolic resin, epoxy resin, acrylic resin, and benzoguanamine resin; and inorganic substances such as silica (such as dimethylsilylated silica), silicates (borosilicate glass (such as barium borosilicate glass), aluminosilicate glass (such as boroaluminosilicate glass, strontium boroaluminosilicate glass, fluoroaluminosilicate glass, or barium aluminosilicate glass)), ceramics, boron nitride, and barium nitride.

Among the above, silica and silicates are preferable as materials for the non-conductive filler, and dimethylsilylated silica and barium aluminosilicate are more preferable.

The first preparation contains a non-conductive filler, and the content of the non-conductive filler with respect to the total mass of the first preparation is preferably 10% by mass or more, more preferably 20% by mass or more, and still more preferably 30% by mass or more.

In a case in which the second preparation contains a non-conductive filler, the second preparation contains a non-conductive filler and the content of the non-conductive filler with respect to the total mass of the second preparation is preferably 10% by mass or more, more preferably 20% by mass or more, and still more preferably 30% by mass or more.

For the kit for preparing a curable composition of the disclosure, it is preferable that in a case in which the first preparation contains a non-conductive filler, the content of the non-conductive filler with respect to the total mass of the first preparation is 10% by mass or more, and in a case in which the second preparation contains a non-conductive filler, the content of the non-conductive filler with respect to the total mass of the second preparation is 10% by mass or more.

### <Additives>

At least one of the first preparation or the second preparation may each independently additives such as a photopolymerization accelerator, a stabilizer (polymerization inhibitor), a colorant, a fluorescent agent, and a UV absorber in the kit for preparing a curable composition of the disclosure.

Known photopolymerization accelerators can be used as the photopolymerization accelerator. Examples thereof include camphorquinone (CQ) and ethyl dimethylaminobenzoate (EDB).

In addition, an antibacterial substance such as cetylpyridinium chloride, benzalkonium chloride, (meth)acryloyloxydodecylpyridinium bromide, (meth)acryloyloxyhexadecylpyridinium chloride, (meth)acryloyloxydecylammonium chloride, or triclosan may be mixed thereinto.

Known dyes and pigments may be mixed into the kit for preparing a curable composition of the disclosure.

The kit for preparing a curable composition of the disclosure is preferably used for dental materials.

Examples of dental materials include, but are not particularly limited to, dental adhesives, dental filling materials, dental sealants (fissure sealants), abutment construction materials, denture base resins, denture base lining materials, resins for dental crown prostheses (hard resins for dental crowns), and dental autopolymerizing resins.

The kit for preparing a curable composition of the disclosure is particularly preferably used for dental adhesive materials.

Examples of dental adhesives include dental adhesive resin cements, orthodontic adhesive materials, adhesive materials for fixing loose teeth, adhesive materials for cavity application, and dental bonding materials, with dental adhesive resin cements being preferred.

Examples of dental filling materials include dental composite resins (including dental self-adhesive composite resins), root canal filling materials, temporary sealing materials, and lining materials.

### <Curable Composition>

The curable composition of the disclosure for a dental material (also simply referred to as "curable composition" in the disclosure) is a mixture of the first preparation and the second preparation in the kit for preparing a curable composition of the disclosure.

In other words, the curable composition of the disclosure contains the first monomer, the second monomer, the organosiloxane containing a polymerizable group and a hydroxyl or alkoxy group, and the polymerization accelerator in the disclosure.

In addition, the curable composition of the disclosure may further contain the above-described components such as a transition metal compound and an organic peroxide.

The curable composition of the disclosure has the above-described configuration, resulting in excellent adhesion to ceramics and adhesion to dentin.

Specific examples, preferred aspects, and the like of monomers, ascorbic acids, organosiloxanes, transition metal compounds, and organic peroxides in the curable composition are the same as with specific examples, preferred aspects, and the like of monomers, ascorbic acids, organosiloxanes, transition metal compounds, and organic peroxides described above.

It is preferable that the curable composition of the disclosure further contains a non-conductive filler.

Specific examples, preferred aspects, and the like of the non-conductive filler in the curable composition are the same as with the non-conductive filler described above.

The curable composition of the disclosure may contain the above-described additives.

### <Cured Product>

The cured product of the disclosure is a cured product of the curable composition of the disclosure or a cured product obtained using the kit for preparing a curable composition of the disclosure.

The cured product of the disclosure can be preferably used as a dental material. In other words, the dental material of the disclosure preferably contains the cured product of the disclosure.

### Examples

The disclosure will be described in detail below with reference to examples, but the disclosure is not limited to these examples. Specific numerical values of the compounding ratio (content ratio), physical property values, parameters, and the like used in the following description can be replaced with the upper limit values (numerical values defined as "equal to or less than" or "less than") or lower limit values (numerical values defined as "equal to or more than" or "more than") of the compounding ratio (content ratio), physical property values, parameters, and the like described in "DESCRIPTION OF EMBODIMENTS" above.

Details of the components used in Examples are as follows.

### (Transition Metal Compound: Copper Complex)

Cu(OAc)₂: Copper acetate

### (Polymerizable Group-Containing Organosilane: Silane Coupling Agent)

3-MPS: 3-Methacryloxypropyl trimethoxysilane; molecular weight of 248.4; viscosity at 25°C of 2.6 mPa·s

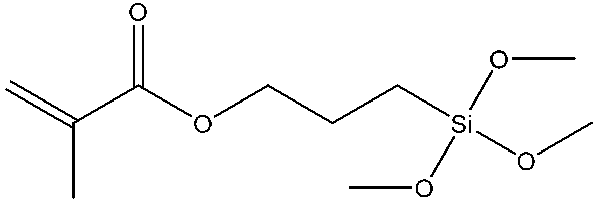

8-MOS: 8-Methacryloxyoctyl trimethoxysilane; molecular weight of 318.5; viscosity at 25°C of 5.3 mPa·s

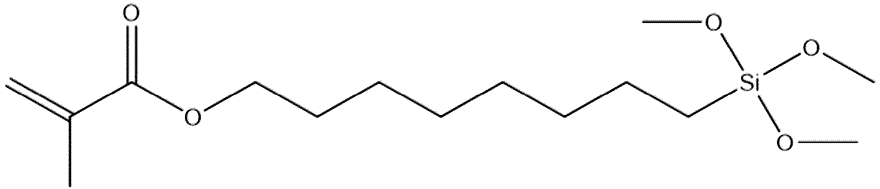

### (Polymerizable Group-Containing Organosiloxane: Silane Coupling Agent)

3-APSO: Siloxane oligomer of 3-acryloxypropyl methoxysilane; molecular weight distribution of from 400 to 800; viscosity at 25°C of 49 mPa·s

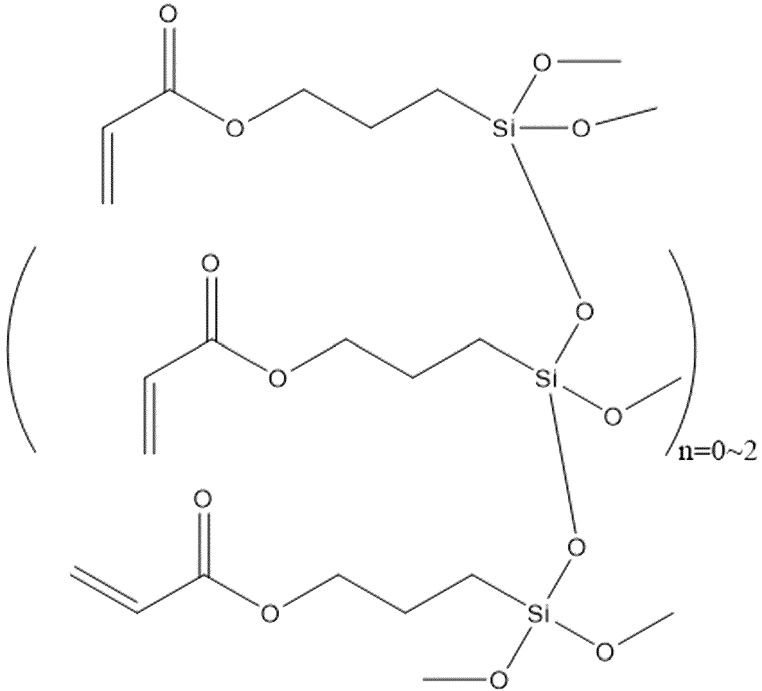

3-MPSO: Siloxane oligomer of 3-methacryloxypropyl methoxysilane; molecular weight distribution of from 400 to 900; viscosity at 25°C of 29 mPa·s

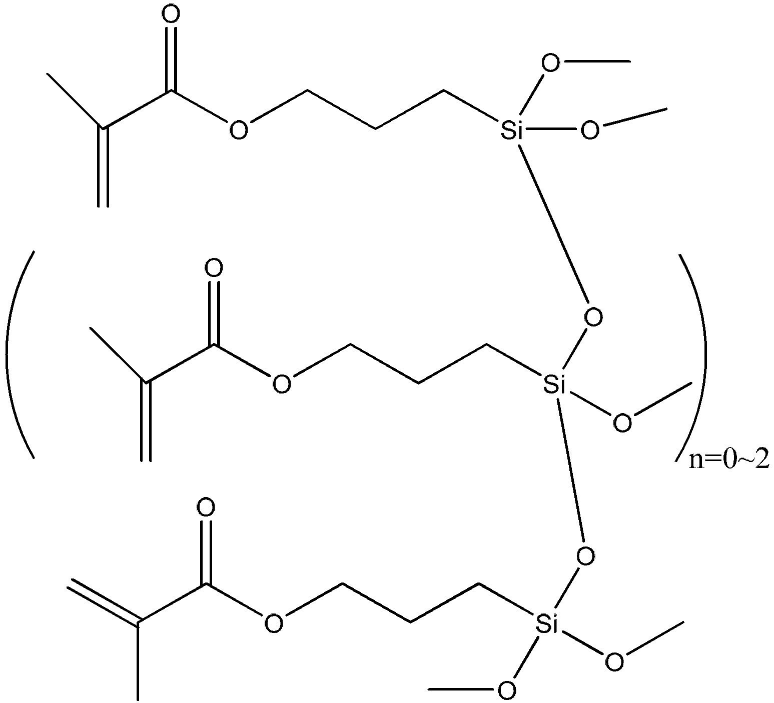

### (Polymerizable Group-Free Organosiloxane)

MMSO1: Siloxane oligomer of methyltrimethoxysilane; molecular weight distribution of from 200 to 700; viscosity at 25°C of 5 mPa·s
MMSO2: Siloxane oligomer of methyltrimethoxysilane; molecular weight distribution of from 200 to 700; viscosity at 25°C of 8 mPa·s
MMSO3: Siloxane oligomer of methyltrimethoxysilane; molecular weight distribution of from 200 to 800; viscosity at 25°C of 29 mPa·s
MMSO4: Siloxane oligomer of methyltrimethoxysilane; molecular weight distribution of rom 200 to 800; viscosity at 25°C of 118 mPa·s

### (Filler)

R812: AEROSIL (registered trademark) R812 manufactured by Evonik Industries AG.
GM27884: GM27884, 1.5 µm manufactured by Schott; amount treated with a silane coupling agent of 2.3%

### (Reducing Agent)

ATH: Acetylthiourea
AACa: Calcium ascorbate
PTS: Sodium salt of p-toluenesulfinic acid
DEPT: Diethanol-p-toluidine
BTA: 1H-Benzotriazole

### (Organic Peroxide)

AHP: tert-Amyl hydroperoxide
tBPB: tert-Butyl perbenzoate

### (Monomer)

UDMA: Trimethylhexyl diurethane hydroxymethacrylate
TEGDMA: Triethylene glycol dimethacrylate
HEMA: 2-Hydroxyethyl methacrylate

### (Acidic Monomer)

MDP: Methacryloxydecyl phosphate

### (Photopolymerization Accelerator)

CQ: Camphorquinone
BEDB: 2-Butoxyethyl-4-(dimethylamino)benzoate

### (Stabilizer)

BHT: 2,6-Di-tert-butyl-p-cresol

### <Kits for Preparing Curable Composition in Examples and Comparative Examples>

The components listed in Tables 1 to 11 were mixed so as to prepare the first and second preparations in each of Examples and Comparative Examples, thereby obtaining kits for preparing a curable composition.

The unit of the content of each component in each table is "% by mass."

### <Ceramic Adhesiveness>

A dental feldspathic glass ceramic (VITA blocs Mark II, manufactured by VITA Zahnfabrik) was placed in a plastic cylindrical container having a diameter of 25 mm and a depth of 25 mm and embedded in an acrylic resin. This was designated as a ceramic adherend. The ceramic adherend was polished with waterproof emery paper (P600) immediately before use and used after grinding off a smooth surface of the ceramic.

An adhesiveness evaluation was carried out in accordance with ISO16506 using, as a test sample used for measuring adhesive strength, separately prepared first and second preparations in each of Examples and Comparative Examples listed in Tables 1 to 4.

Specifically, the first and second preparations in each of Examples and Comparative Examples listed in Tables 1 to 4 were mixed for 20 seconds and applied in an appropriate amount to a cylindrical cured product (Venus Diamond manufactured by Kulzer; the adhering surface was polished in advance with waterproof emery paper P600) of a filler composition prepared in advance, which was then placed vertically onto the ceramic adherend prepared as described above. Thereafter, a specialized tool was used to apply a pressure of 5N.

After removing the excess of the applied composition, in the case of chemical polymerization, the test sample was left to stand at 37°C for 40 minutes while attached to the specialized tool, and then left to stand at 37°C for 90 minutes with the tool removed. The test sample was then immersed in distilled water at 37°C and allowed to stand in a thermostatic chamber at 37°C for 24 hours, after which the adhesive strength was measured.

The adhesive strength was determined by applying a shear load parallel to and in contact with the surface of the ceramic adherend at a crosshead speed of 1.0 mm/min, and measuring the shear load at which the cylindrical cured product adhering to the ceramic adherend was peeled off from the surface.

The obtained adhesive strength was evaluated according to the following evaluation criteria and used as an index of adhesiveness to feldspar ceramic.

### Evaluation Criteria

A: After the test, the fracture of the ceramic adherend was observed over 80% or more of the adhesion surface area (also referred to as material fracture in the disclosure).
B: After the test, the fracture of the ceramic adherend was observed over from 20% to less than 80% of the adhesion surface area, and interfacial peeling was observed between the adhesive cement (i.e., the cured product of the mixture of the first and second preparations) and the ceramic adherend (also referred to as mixed fracture in the disclosure).
C: After the test, the interfacial peeling between the adhesive cement and the ceramic adherend was observed over more than 80% of the adhesion surface area (also referred to as interfacial fracture in the disclosure).

The occurrence of interfacial peeling between the ceramic adherend and the adhesive cement indicates poor adhesion to the ceramic. The occurrence of the fracture of the ceramic adherend indicates the strength of the adhesion to the ceramic.

For example, in the material fracture in the above test, mainly, the interfacial peeling between the ceramic adherend and the adhering cement does not occur, but the fracture of the ceramic adherend occurs. Therefore, among A to C above, A corresponds to the most excellent adhesion to the ceramic adherend.

Meanwhile, during interfacial fracture, mainly the fracture of the ceramic adherend does not occur, but the interfacial peeling between the ceramic adherend and the adhesive cement occurs in the above-described test. Therefore, among A to C above, C corresponds to the poorest adhesion to the ceramic adherend.

### <Measurement of Fracture Strength>

A three-point bending test was carried out in accordance with ISO16506 to measure the fracture strength of each cured product.

Specifically, the first and second preparations in each of Examples and Comparative Examples listed in Tables 1 to 4 were mixed for 20 seconds, placed in a stainless steel mold of 2 mm × 2 mm × 25 mm in size, allowed to stand in a thermostatic chamber at 37°C for 10 minutes, and then immersed in distilled water at 37°C for 50 minutes. Further, the cured product was removed from the stainless steel mold, immersed in distilled water at 37°C, and left at 37°C for 24 hours, which was then used as a test piece for the bending test.

The test piece prepared by the above-described method was subjected to a three-point bending test using a testing machine (Autograph EZ-S manufactured by Shimadzu Corporation) with a support distance of 20 mm and a crosshead speed of 1 mm/min for measuring the breaking strength.

**[Table 1]**

| | | Comparative Example 1 | | Comparative Example 2 | | Comparative Example 3 | | Example 1 | | Example 2 | |
|---|---|---|---|---|---|---|---|---|---|---|---|
| | | 1st preparation | 2nd preparation | 1st preparation | 2nd preparation | 1st preparation | 2nd preparation | 1st preparation | 2nd preparation | 1st preparation | 2nd preparation |
| Monomer not having acidic monomer | UDMA | 19 | 20 | 19 | 20 | 19 | 20 | 19 | 20 | 19 | 20 |
| | TEGDMA | 10 | 15 | 10 | 9 | 10 | 9 | 10 | 9 | 10 | 9 |
| | HEMA | 3 | | 3 | | 3 | | 3 | | 3 | |
| Acidic monomer | MDP | 10 | | 10 | | 10 | | 10 | | 10 | |
| Organosilane having a polymerizable group and a hydroxyl or alkoxy group | 3-MPS | | | | 6 | | | | | | |
| | 8-MOS | | | | | | 6 | | | | |
| Organosiloxane having a polymerizable group and a hydroxyl or alkoxy group | 3-APSO | | | | | | | | 6 | | |
| | 3-MPSO | | | | | | | | | | 6 |
| Stabilizer | BHT | 0.05 | 0.05 | 0.05 | 0.05 | 0.05 | 0.05 | 0.05 | 0.05 | 0.05 | 0.05 |
| Reducing agent | ATH | | 1.5 | | 1.5 | | 1.5 | | 1.5 | | 1.5 |
| | AACa | | | | | | | | | | |
| | PTS | | | | | | | | | | |
| | DEPT | | | | | | | | | | |
| | BTA | | | | | | | | | | |
| Transition metal complex | 1% Cu(OAc)₂ in UDMA | 1 | | 1 | | 1 | | 1 | | 1 | |
| Organic peroxide | AHP | 1 | | 1 | | 1 | | 1 | | 1 | |
| | tBP8 | | | | | | | | | | |
| Photopolymerization accelerator | CQ | | 0.1 | | 0.1 | | 0.1 | | 0.1 | | 0.1 |
| | BEDB | 0.1 | | 0.1 | | 0.1 | | 0.1 | | 0.1 | |
| Filler | R812 | 6 | 3 | 6 | 3 | 6 | 3 | 6 | 3 | 6 | 3 |
| | GM27884 | 50 | 60 | 50 | 60 | 50 | 60 | 50 | 60 | 50 | 60 |
| Adhesion test | Adhesion to feldspathic glass ceramic | C | | A | | A | | A | | A | |
| Three-point bending test | Fracture strength(MPa) | 71 | | 53 | | 57 | | 72 | | 67 | |
| | Fracture strength ratio to Comparative Example 1 (%) | - | | 74.6 | | 80.3 | | 101.4 | | 94.4 | |

**[Table 2]**

| | | Comparative Example 4 | | Comparative Example 5 | | Comparative Example 6 | | Comparative Example 7 | |
|---|---|---|---|---|---|---|---|---|---|
| | | 1st preparation | 2nd preparation | 1st preparation | 2nd preparation | 1st preparation | 2nd preparation | 1st preparation | 2nd preparation |
| Monomer not having acidic monomer | UDMA | 19 | 20 | 19 | 20 | 19 | 20 | 19 | 20 |
| | TEGDMA | 10 | 9 | 10 | 9 | 10 | 9 | 10 | 9 |
| | HEMA | 3 | | 3 | | 3 | | 3 | |
| Acidic monomer | MDP | 10 | | 10 | | 10 | | 10 | |
| Organosiloxane not having polymerizable group but having hydroxyl or alkoxy group | MMSO1 | | 6 | | | | | | |
| | MMSO2 | | | | 6 | | | | |
| | MMSO3 | | | | | | 6 | | |
| | MMSO4 | | | | | | | | 6 |
| Stabilizer | BHT | 0.05 | 0.05 | 0.05 | 0.05 | 0.05 | 0.05 | 0.05 | 0.05 |
| Reducing agent | ATH | | 1.5 | | 1.5 | | 1.5 | | 1.5 |
| | AACa | | | | | | | | |
| | PTS | | | | | | | | |
| | DEPT | | | | | | | | |
| | BTA | | | | | | | | |
| Transition metal complex | 1%Cu(OAc)₂ in UDMA | 1 | | 1 | | 1 | | 1 | |
| Organic peroxide | AHP | 1 | | 1 | | 1 | | 1 | |
| | tBPB | | | | | | | | |
| Photopolymerization accelerator | CQ | | 0.1 | | 0.1 | | 0.1 | | 0.1 |
| | BEDB | 0.1 | | 0.1 | | 0.1 | | 0.1 | |
| Filler | R812 | 6 | 3 | 6 | 3 | 6 | 3 | 6 | 3 |
| | GM27884 | 50 | 60 | 50 | 60 | 50 | 60 | 50 | 60 |
| Adhesion test | Adhesion to feldspathic glass ceramic | C | | C | | C | | C | |
| Three-point bending test | Fracture strength(MPa) | 59 | | 52 | | 55 | | 61 | |
| | Fracture strength ratio to Comparative Example 1 (%) | 83.1 | | 73.2 | | 77.5 | | 85.9 | |

**[Table 3]**

| | | Comparative Example 8 | | Example 3 | | Example 4 | |
|---|---|---|---|---|---|---|---|
| | | 1st preparation | 2nd preparation | 1st preparation | 2nd preparation | 1st preparation | 2nd preparation |
| Monomer not having acidic monomer | UDMA | 18 | 20 | 18 | 20 | 18 | 20 |
| | TEGDMA | 10 | 15 | 10 | 9 | 10 | 9 |
| | HEMA | 3 | | 3 | | 3 | |
| Acidic monomer | MDP | 10 | | 10 | | 10 | |
| Organosilane having a polymerizable group and a hydroxyl or alkoxy group | 3-MPS | | | | | | |
| | 8-MOS | | | | | | |
| Organosiloxane having a polymerizable group and a hydroxyl or alkoxy group | 3-APSO | | | | 6 | | |
| | 3-MPSO | | | | | | 6 |
| Stabilizer | BHT | 0.05 | 0.05 | 0.05 | 0.05 | 0.05 | 0.05 |
| Reducing agent | ATH | | | | | | |
| | AACa | | 1.5 | | 1.5 | | 1.5 |
| | PTS | | | | | | |
| | DEPT | | | | | | |
| | BTA | | | | | | |
| Transition metal complex | 1% Cu(OAc)₂ in UDMA | 2 | | 2 | | 2 | |
| Organic peroxide | AHP | 1 | | 1 | | 1. | |
| | tBPB | | | | | | |
| Photopolymerization accelerator | CQ | | 0.1 | | 0.1 | | 0.1 |
| | BEDB | 0.1 | | 0.1 | | 0.1 | |
| Filler | R812 | 6 | 3 | 5 | 3 | 5 | 3 |
| | GM27884 | 50 | 60 | 50 | 60 | 50 | 60 |
| Adhesion test | Adhesion to feldspathic glass ceramic | C | | A | | A | |
| Three-point bending test | Fracture strength(MPa) | 102 | | 104 | | 100 | |
| | Fracture strength ratio to Comparative Example 8 (%) | - | | 102.0 | | 98.0 | |

**[Table 4]**

| | | Comparative Example 9 | | Example 5 | | Example 6 | |
|---|---|---|---|---|---|---|---|
| | | 1st preparation | 2nd preparation | 1st preparation | 2nd preparation | 1st preparation | 2nd preparation |
| Monomer not having acidic monomer | UDMA | 20 | 20 | 20 | 20 | 20 | 20 |
| | TEGDMA | 10 | 15 | 10 | 9 | 10 | 9 |
| | HEMA | 3 | | 3 | | 3 | |
| Acidic monomer | MDP | 10 | | 10 | | 10 | |
| Organosilane having a polymerizable group and a hydroxyl or | 3-MPS | | | | | | |
| alkoxy group | 8-MOS | | | | | | |
| Organosiloxane having a polymerizable group and a hydroxyl or alkoxy group | 3-APSO | | | | 6 | | |
| | 3-MPSO | | | | | | 6 |
| Stabilizer | BHT | 0.05 | 0.05 | 0.05 | 0.05 | 0.05 | 0.05 |
| Reducing agent | ATH | | | | | | |
| | AACa | | | | | | |
| | PTS | | 1 | | 1 | | 1 |
| | DEPT | | 0,2 | | 0,2 | | 0,2 |
| | BTA | | 1 | | 1 | | 1 |
| Transition metal complex | 1% Cu(OAc)₂ in UDMA | 0.5 | | 0.5 | | 0.5 | |
| Organic peroxide | AHP | | | | | | |
| | tBPB | 0.2 | | 0.2 | | 0.2 | |
| Photopolymerization accelerator | CQ | | 0.1 | | 0.1 | | 0.1 |
| | BEDB | 0.1 | | 0.1 | | 0.1 | |
| Filler | R812 | 6 | 3 | 6 | 3 | 6 | 3 |
| | GM27884 | 50 | 60 | 50 | 60 | 50 | 60 |
| Adhesion test | Adhesion to feldspathic glass ceramic | B | | A | | A | |
| Three-point bending test | Fracture strength(MPa) | 99 | | 117 | | 104 | |
| | Fracture strength ratio to Comparative Example 9 (%) | - | | 118.2 | | 105.1 | |

As shown in each table, the results in the adhesion test were excellent, and the decrease in fracture strength was suppressed in Examples, in which a kit for preparing a curable composition for a dental material including a polymerization accelerator, the kit including a first preparation including a first monomer and a second preparation including a second monomer, in which at least one of the first preparation or the second preparation contained an organosiloxane including a polymerizable group and a hydroxyl or alkoxy group, was used. Therefore, it was possible to obtain a cured product that is excellent in adhesion to ceramics and is suppressed in the decrease of mechanical properties in Examples.

Meanwhile, the cured product of Comparative Example 1, which did not contain an organosiloxane, had poor adhesiveness.

The cured products of Comparative Examples 2 and 3, which contained an organosilane but not an organosiloxane, had a decrease in fracture strength compared with Comparative Example 1.

The cured products of Comparative Examples 4 to 7, which contained an organosiloxane but did not contain a polymerizable group in it, were inferior in adhesiveness and had reduced fracture strength.

Meanwhile, the cured products of Comparative Examples 8 and 9, which did not contain an organosiloxane, had poor adhesiveness.

The disclosure of Japanese Patent Application No. 2022-124315, filed on August 3, 2022, is incorporated herein by reference in its entirety.

All publications, patent applications, and standards mentioned herein are incorporated herein by reference to the same extent as if each individual publication, patent application, or standard was specifically and individually indicated to be incorporated by reference.

## Claims

1. A kit for preparing a curable composition for a dental material including a polymerization accelerator, the kit comprising:
a first preparation containing a first monomer; and
a second preparation containing a second monomer,
wherein at least one of the first preparation or the second preparation contains an organosiloxane containing a polymerizable group and a hydroxyl or alkoxy group.

2. The kit for preparing a curable composition for a dental material according to claim 1, wherein the organosiloxane has a molecular weight of from 200 to 1500.

3. The kit for preparing a curable composition for a dental material according to claim 1, wherein the polymerization accelerator contains an oxidant and a reducing agent.

4. The kit for preparing a curable composition for a dental material according to claim 3, wherein:
the oxidant contains an organic peroxide,
the reducing agent contains a reducing agent (A) that is a transition metal compound and a reducing agent (B) other than the transition metal compound,
the first preparation contains the organic peroxide and the reducing agent (A), and
the second preparation contains the reducing agent (B).

5. The kit for preparing a curable composition for a dental material according to claim 4, wherein the reducing agent (B) contains at least one selected from the group consisting of a thiourea, an ascorbic acid, a sulfinic acid, and an amine compound.

6. The kit for preparing a curable composition for a dental material according to claim 1, wherein either one of the first monomer or the second monomer contains an acidic monomer.

7. The kit for preparing a curable composition for a dental material according to claim 6, wherein the first monomer contains the acidic monomer.

8. The kit for preparing a curable composition for a dental material according to claim 1, wherein the first preparation and the second preparation contain a filler.

9. The kit for preparing a curable composition for a dental material according to claim 1, wherein a total content of the organosiloxane contained in the first preparation and the second preparation is from 0.1% by mass to 15.0% by mass with respect to a total mass of a curable composition for a dental material that is to be prepared.

10. A curable composition for a dental material, comprising a mixture of the first preparation and the second preparation of the kit for preparing a curable composition for a dental material according to claim 1.

11. A dental material, comprising a cured product of the curable composition for a dental material according to claim 10.
